Europäisches Patentamt

European Patent Office

Office européen des brevets

(11) Veröffentlichungsnummer: **0 577 973 A2**

# (12) EUROPÄISCHE PATENTANMELDUNG

(21) Anmeldenummer: 93108913.0

(22) Anmeldetag: 03.06.93

(51) Int. Cl.5: **C12N 9/54**, C11D 3/386, C12N 1/20, //(C12N1/20, C12R1:07)

(30) Priorität: 11.06.92 DE 4219104

(43) Veröffentlichungstag der Anmeldung: **12.01.94 Patentblatt 94/02**

(84) Benannte Vertragsstaaten: **AT BE CH DE DK ES FR GB GR IE IT LI LU NL PT SE**

(71) Anmelder: **Solvay Enzymes GmbH & Co. KG Grosse Drakenburger Strasse 95 D-31582 Nienburg(DE)**

(72) Erfinder: **Vetter Roman Warneckeweg 1 W-3167 Burgdorf(DE)**
Erfinder: **Möller, Bernhard Neuweilerstrasse 12 D-52477 Alsdorf(DE)**
Erfinder: **Preen, Klaus Friedrichstrasse 2 W-3257 Springe(DE)**
Erfinder: **Mücke, Ingo Knickstrasse 3a W-3013 Barsinghausen(DE)**
Erfinder: **Konieczny-Janda, Gerhard Schöneberger Strasse 23 W-3017 Pattensen(DE)**

(74) Vertreter: **Lauer, Dieter, Dr. c/o Solvay Deutschland GmbH Hans-Böckler-Allee 20 D-30173 Hannover (DE)**

(54) **Alkalische Protease aus Bacillus sp. MF12, ein Verfahren zu deren Herstellung und deren Verwendung.**

(57) Beschrieben wird eine alkalische Bacillus-Protease aus Bacillus sp. MF12, deren Verwendung und ein Verfahren zur Herstellung dieser Protease. Die erfindungsgemäße alkalische Protease eignet sich für die Anwendung in Zusammensetzungen für Reinigungs- und Waschzwecke.

EP 0 577 973 A2

Die vorliegende Erfindung betrifft eine alkalische Protease aus Bacillus sp. MF12, deren Verwendung und ein Verfahren zur Herstellung dieser Protease.

Alkalische Proteasen sind wertvolle industrielle Produkte mit vorteilhaften Anwendungen, insbesondere in der Waschmittelindustrie, da sie proteinenthaltende Verunreinigungen entfernen. Um wirksam zu sein, müssen diese Proteasen nicht nur proteolytische Aktivität unter Waschbedingungen (pH-Wert, Temperatur) besitzen, sondern sie müssen darüber hinaus auch mit anderen Waschmittelbestandteilen, d.h. in Kombination mit anderen Enzymen, Tensiden, Gerüststoffen (Builder), Bleichmitteln, Bleichmittelaktivatoren und anderen Zusatz- und Hilfsstoffen verträglich sein. Insbesondere müssen die Proteasen gegenüber diesen Waschmittelbestandteilen ausreichende Stabilität und in deren Gegenwart ausreichende Waschwirksamkeit aufweisen.

Die alkalischen Proteasen des Standes der Technik werden bisher insbesondere durch Kultivierung von Bacillus-Spezies wie z.B. Bacillus alcalophilus, Bacillus subtilis, Bacillus amyloliquefaciens und Bacillus licheniformis, die alkalische Proteasen produzieren und in das Kulturmedium ausscheiden, erhalten.

In Bezug auf alkalische Proteasen wurden im Stand der Technik bereits viele Anstrengungen unternommen, um neue alkalische Proteasen mit gewünschten Eigenschaften zu erhalten. Dennoch besteht Bedarf an neuen, alkalischen Proteasen mit insbe sondere im Hinblick auf das Waschverhalten günstigen Eigenschaften.

Es bestand daher die Aufgabe, eine neue wertvolle alkalische Protease mit günstigen Eigenschaften herzustellen.

Überraschenderweise wurde nunmehr gefunden, daß die alkalische Bacillus-Protease, die durch Kultivierung von Bacillus sp. MF12 DSM 6845 erhältlich ist, vorteilhafte Eigenschaften besitzt und sehr gute Waschwirksamkeit zeigt.

Gegenstand der Erfindung ist daher eine alkalische Bacillus-Protease mit einem pH-Optimum im pH-Bereich von etwa pH 9,5 bis 13,0 und einem Temperatur-Optimum im Bereich von etwa 55 bis 65 °C, die durch Kultivierung von Bacillus sp. MF12 DSM 6845 erhältlich ist.

Bevorzugt umfaßt die Erfindung dabei eine alkalische Protease aus Bacillus sp. MF12 DSM 6845 mit den folgenden Eigenschaften:

(1) Wirkung: Abbau von Proteinen und Peptiden;

(2) pH-Optimum: etwa bei pH-Werten von 9,5 bis 13,0;

(3) pH-Stabilität: bei pH-Werten von 9,5 bis 11,0 erweist sich die Protease als völlig stabil;

(4) Temperatur-Optimum: etwa 64 °C,

(5) Temperatur-Stabilität: durch Inkubation der Protease bei Temperaturen bis 30 °C für 15 Minuten wird die Protease nicht wesentlich in ihrer Aktivität beeinträchtigt; nach 15 Minuten Inkubation bei etwa 40 °C beträgt die Restaktivität der Protease wenigstens 75 %.

Die erfindungsgemäße Bacillus-Protease eignet sich als Additiv für Waschmittel- und Reinigungsmittelzusammensetzungen etc., die neutrale bis alkalische pH-Werte besitzen und bei niedrigen Temperaturen, insbesondere bei Temperaturen bis 60 °C angewendet werden. Die Erfindung betrifft daher auch die Verwendung der erfindungsgemäßen alkalischen Bacillus-Protease in Waschmittel-, Reinigungsmittel- oder Geschirrspülmittelzusammensetzungen. Sie kann hierbei auch vorteilhaft in Gegenwart von anderen üblichen Enzymen, insbesondere auch in Gegenwart von anderen Proteasen, eingesetzt werden. Eine besonders bevorzugte Verwendung der erfindungsgemäßen alkalischen Protease betrifft deren Verwendung in Waschmittel-, Reinigungsmittel- oder Geschirrspülmittelzusammensetzungen für niedrige Anwendungstemperaturen bis 60 °C.

Ein weiterer Gegenstand der Erfindung sind Waschmittel- und Reinigungsmittelzusammensetzungen, wie z.B. Textil- oder Geschirreinigungsmittel, die die erfindungsgemäße alkalische Protease enthalten. Für diese Anwendungszwecke stellt die Erfindung eine neue alkalische Protease zur Verfügung, die ihre günstigen waschwirksamen Eigenschaften in vorteilhafter Weise in den vorgenannten Zusammensetzungen entfalten kann. Die erfindungsgemäße Protease kann in Wasch- und Reinigungsmittelformulierungen, beispielsweise in Flüssig- oder Pulverwaschmittelformulierungen, einzeln oder gegebenenfalls auch in Kombination mit Wasch- und Reinigungsmittelproteasen des Standes der Technik oder anderen in solchen Zusammensetzungen üblichen Enzymen, wie z.B. Proteasen, Amylasen, Lipasen, Pektinasen, Nukleasen, Oxidoreduktasen, Cellulasen etc., eingesetzt werden. Die erfindungsgemäße Protease wird in den Wasch- und Reinigungsmittelformulierungen in an sich für Waschmittelenzyme üblichen Mengen, insbesondere in Mengen von bis zu 3 Gew.-% (bezogen auf die Trockensubstanz der Gesamtzusammensetzung), vorzugsweise in einer Menge von 0,2 bis 1,5 Gew.-%, verwendet.

Außer den bereits erwähnten Waschmittelenzymen können die Wasch- und Reinigungsmittel der Erfindung alle an sich im Stand der Technik üblichen Waschmittelinhaltsstoffe wie Tenside, Bleichmittel oder Gerüststoffe (Builder), sowie weitere übliche Hilfsstoffe für die Formulierung von Waschmitteln in an sich üblichen Mengen enthalten. Zu den Hilfsstoffen gehören z.B. Verstärker, Enzymstabilisatoren, Schmutzträger und/oder Kompatibilisierungsmittel, Komplex- und Chelatbildner, Seifenschaumregulatoren und Zusatzstoffe wie optische Aufheller, Opazifizierungsmittel, Korrosionsinhibitoren, Antielektrostatika, Farbstoffe, Bakterizide, Bleichmittelaktivatoren, Persäurebleichmittelvorstufen.

So enthalten erfindungsgemäße Waschmittelformulierungen in typischer beispielhafter Zusammensetzung bezogen auf Trockensubstanz

a) wenigstens 5 Gew.-%, z.B. 10 bis 50 Gew.-%, eines Tensids oder Tensidgemisches

b) bis zu 40 Gew.-% eines Builders oder eines Builder-Gemisches,

c) bis zu 40 Gew.-% eines Bleichmittels oder Bleichmittelgemisches, vorzugsweise ein Perborat wie Natriumperborat-Tetrahydrat oder Natriumperborat-Monohydrat,

d) bis zu 3 Gew.-% der erfindungsgemäßen Protease

e) weitere Bestandteile wie Hilfsstoffe etc. ad 100 Gew.-%

Solche Waschmittelformulierungen können in an sich üblicher Weise formuliert werden. Die erfindungsgemäße Protease kann dazu z.B. in Form von Granulaten, Prills oder Pellets, gegebenenfalls auch mit Oberflächenüberzügen versehen, mit den anderen Komponenten der Waschmittelformulierung in an sich bekannter Weise vermischt werden.

Die erfindungsgemäße Protease eignet sich auch sehr gut für den Einsatz in an sich üblichen Flüssigwaschmittelformulierungen.

Weiterhin umfaßt die Erfindung ein Verfahren zur Herstellung der erfindungsgemäßen alkalischen Protease. So wird die erfindungsgemäße alkalische Protease erhalten, indem man den Bacillus sp. MF12 mit der Hinterlegungsnummer DSM 6845 kultiviert und nachfolgend aus dem Kulturüberstand die gebildete alkalische Protease isoliert. Die Isolierung der Protease aus dem Kulturüberstand wird dabei durchgeführt, indem man die Bakterienzellen durch Zentrifugation oder Filtration abtrennt. Gegebenenfalls kann aus dem verbleibenden Kulturüberstand die Protease durch Membranfiltration oder Fällung weiter konzentriert und aufgereinigt werden.

Die erfindungsgemäße Bacillus sp. MF12-Protease zeichnet sich durch vorteilhafte Eigenschaften aus. Sie besitzt bei alkalischen pH-Werten eine hohe Stabilität. Das pH-Optimum der erfindungsgemäßen Proteasen liegt in einem für die Anwendung in Wasch- und Reinigungsmittelzusammensetzungen günstigen Bereich. Ferner besitzt die erfindungsgemäße Protease ein Temperatur-Optimum im Bereich von etwa 55 bis 65 °C. Ferner zeigt sie gute Stabilitäten auch in Waschlauge. Aufgrund ihrer günstigen Aktivität bei Temperaturen bis zu 65 °C eignet sich die erfindungsgemäße Bacillus-Protease insbesondere für die Anwendung in Reinigungs- und Waschmittelzusammensetzungen, die bei niedrigen Temperaturen, insbesondere bis 60 °C, angewendet werden sollen. Solche Wasch- und Reinigungsmittelzusammensetzungen, die eine erfindungsgemäße Protease enthalten, zeigen günstige Waschwirksamkeit hinsichtlich zu entfernender Proteinanschmutzungen. Besonders vorteilhaft ist es, daß dabei auch unterschiedliche Arten an Proteinverschmutzungen beim Waschgang durch die erfindungsgemäße Protease aus dem Gewebe entfernt werden.

Die nachfolgende Offenbarung gibt zur weiteren Erläuterung der Erfindung typische beispielhafte Ausgestaltungen der Erfindung wieder, ohne jedoch die Erfindung in ihrem Umfange zu beschränken.

Bei der Angabe von Enzymstabilitäten in den folgenden Beispielen bedeuten: "völlig stabil" eine Restaktivität von mindestens 90 %; "stabil" eine Restaktivität von wenigstens 80 %.

Der hier verwendete, im Beispiel 1 und der Beschreibung angegebene Bacillus sp. MF12-Stamm wurde aus der Natur isoliert und bei der Deutschen Sammlung von Mikroorganismen (DSM) unter der Nummer DSM 6845 am 16.12.1991 hinterlegt.

Erläuterungen zu den Figuren:

Figur 1:
Temperatur-Optimum der Protease aus Bacillus sp. MF12 (DSM 6845).
Figur 2:
Temperatur-Stabilität der Protease aus Bacillus sp. MF12 (DSM 6845).
Figur 3:
pH-Optimum der Protease aus Bacillus sp. MF12 (DSM 6845).
Figur 4:
pH-Stabilität der Protease aus Bacillus sp. MF12 (DSM 6845). Zur pH-Wert-Einstellung wurde für den pH-Bereich pH 5 bis pH 7 0,1 M Phosphatpuffer, für den Bereich pH 7 bis pH 9 0,1 M Tris-HCl-Puffer und für den Bereich pH 9 bis pH 12,1 0,1 M Glycin-NaOH-Puffer benutzt.

Beispiel 1

Isolierung eines Bacillus sp. MF12 aus der Natur und dessen Identifizierung.

Der Stamm Bacillus sp. MF12 wurde aus der Natur isoliert und bei der Deutschen Sammlung für Mikroorganismen unter der Nummer DSM 6845 hinterlegt. Es handelt sich um einen grampositiven, sporenbildenden, fakultativ aeroben Mikroorganismus der Gattung Bacillus. Die zell- und koloniemorphologische Beschreibung wird im folgenden gegeben, biochemische Reaktionen und Reaktionen auf bestimmte Wuchsbedingungen sind in der Tabelle 1 aufgeführt.

Bacillus sp. MF12 DSM 6845:

Stäbchenförmiges Bakterium mit abgerundeten Ecken. Die Gramreaktion (L-Aminopeptidase-Test, KOH-Test) ist positiv. Auf P8A-Agar (siehe unten) haben die Kolonien nach 42 Stunden bei 37 °C einen Durchmesser von 2 bis 3 mm, sind gelb-beigefarben, opaque, flach, kreisförmig bis unregelmäßig mit einem gelappten Rand. Die Zellen haben auf LB/CO$_3$-Agar einen Durchmesser von 6 bis 10 mm und sind gelb-beige, opaque, flach, kreisförmig bis unregelmäßig mit gelappten Rand. Die Zellen haben nach 22-stündigem Wachstum in P8A-Nährlösung bei 37 °C und 120 Upm eine Größe von 0,76 bis 0,95 $\mu$m x 1,9 bis 3,8 $\mu$m. Die Sporen sind oval, das Sporangium ist geschwollen.

| LB/CO$_3$-Agar: | |
|---|---|
| Hefeextrakt | 10 g |
| Trypton | 5 g |
| NaCl | 5 g |
| Carbonat-Puffer | 50 ml |
| Agar | 20 g |
| Aqua bidest. | ad 1000 ml |
| pH | 9,5 |

| Carbonat-Puffer: | |
|---|---|
| Na$_2$CO$_3$ | 53 g |
| NaHCO$_3$ | 42 g |
| Aqua bidest. | ad 1000 ml |

| Magermilch-Lösung: | |
|---|---|
| Magermilch (Skim milk) | 100 g |
| Aqua bidest. | ad 1000 ml |

| P8A-Agar: | |
|---|---|
| Agar | 20 g |
| P8A-Nährlösung | ad 1000 ml |

4

EP 0 577 973 A2

| P8A-Nährlösung: | |
|---|---|
| Nutrient Broth | 8 g |
| KCl | 1 g |
| $CaCl_2 \cdot 2H_2O$ | 0,2 g |
| $MgSo_4 \cdot 7H_2O$ | 40 mg |
| $MnCl_2 \cdot 4H_2O$ | 1 mg |
| $FeCl_3$ | 8 mg |
| Magermilch-Lösung | 100 ml |
| Carbonat-Puffer | 50 ml |
| Aqua bidest. | ad 1000 ml |

Es wurden die in Tabelle 1 aufgeführten Tests nach Bergeys Manual of Systematic Bacteriology, Vol.2, 1121-1125, P.H.A. Sneath (ed.), Williams und Wilins, Baltimore-London-Los Angeles-Sydney, 1986 durchgeführt.

Eine genaue Zuordnung zu einer Bacillus-Art war nicht möglich. Der Stamm erhielt die Bezeichnung Bacillus sp. MF12.

Tabelle 1

Charakteristische Merkmale des Bacillus sp. MF12 (DSM6845)

| Merkmal | MF 12 |
|---|---|
| Zelldurchmesser > 1 μm | – |
| Sporenform rund | – |
| Sporangium geschwollen | + |
| Parasporale Kristalle | – |
| Catalase | + |
| Anaerobes Wachstum | + |
| Voges–Proskauer Test | – |
| pH in V–P Nährlösung | |
| < 6 | – |
| > 7 | + |
| Säurebildung auf | |
| Glucose | + |
| L–Arabinose | + |
| D–Xylose | + |
| D–Mannitol | + |
| Gasbildung mit Glucose | – |
| Hydrolyse von | |
| Casein | + |
| Gelatine | + |
| Stärke | + |
| Metabolisierung von | |
| Citrat | – |
| Propionat | – |
| Abbau von Tyrosin | – |
| Desaminierung von Phenylalanin | + |
| Eigelb–Lecithinase | + |
| Nitratreduktion zu Nitrit | + |
| Bildung von | |
| Indol | – |
| Dihydroxyaceton | + |
| NaCl– u. KCl–Bedürfnis | n.b. |
| Allantoin oder ureate required | n.b. |
| Wachstum bei pH | |
| 6.8 in NB | – |
| 5.7 | – |
| Wachstum in NaCl | |
| 2 % | n.b. |
| 5 % | + |
| 7 % | + |
| 10 % | + |
| Wachstum bei | |
| 5°C | – |
| 10°C | n.b. |
| 30°C | + |
| 40°C | n.b. |
| 50°C | n.b. |
| 55°C | n.b. |
| 65°C | n.b. |
| Wachstum in Gegenwart von Lysozyme | + |
| Autotroph mit H3 + CO2 oder CO | n.b. |

Bestimmung der Merkmale nach Bergey's Manual of Systematic Bacteriology, Vol.2, S. 1123, P.H.A. Sneath, ed., Williams u. Wilkins, Baltimore-London-Los Angeles-Sydney, 1986. (+, 90 % oder mehr positiv; – 90 % oder mehr negativ; n.b., nicht bestimmt; NB, Nährbouillon)

Beispiel 2

Herstellung der alkalischen Protease aus Bacillus sp. MF12 DSM 6845.

50 ml Luria-Broth pH 9,5 (10 g Hefe-Extrakt, 5 g Trypton, 5 g NaCl und 50 ml Carbonat-Puffer ad 1000 ml Aqua bidest.) in 500 ml Erlenmeyerkolben mit 3 Schikanen wurden mit einer Einzelkolonie des Stammes Bacillus sp. MF12 (gewachsen auf P8A-Agar-Platten) beimpft und für 16 Stunden bei 37 °C und 240 Upm inkubiert. Mit 2,5 ml dieser Kultur wurden 50 ml Hauptkultur Medium (Soja 2 %; Stärke 5 %; Maisquellwasser 1 %, Carbonatpuffer 50 ml ($Na_2CO_3$ 4,2 %)) in 500 ml-Erlenmeyerkolben mit 3 Schikanen beimpft und für 48 Stunden bei 37 °C und 240 Upm inkubiert. Nach 48 Stunden betrug die proteolytische Aktivität im Kulturüberstand, gewonnen durch Zentrifugation für 15 Minuten bei 27 000 x g, 5000 Delft Units/ml (Definition "Delft Unit" siehe Beispiel 3).

Beispiel 3

Bestimmung der Enzymcharakteristika der Bacilllus sp. MF12-Protease.

Die Aktivität der Protease wurde in Delft Units (DU) bestimmt. 1000 DU ist die proteolytische Aktivität, die bei einem Volumen von 1 ml einer 2 % (w/w)igen Enzymlösung nach Abbau von Casein eine Extinktionsdifferenz (1 cm Lichtweg; 275 nm; Bestimmung gegen Blindprobentest) von 0,4000 ergibt.

Zur Bestimmung der Enzymcharakteristika wie Temperatur-Optimum, Temperatur-Stabilität, pH-Optimum und pH-Stabilität wurde der im Beispiel 2 durch Kultivierung des Bacillus sp. MF12 DSM 6845 erhaltene Kulturüberstand verwendet.

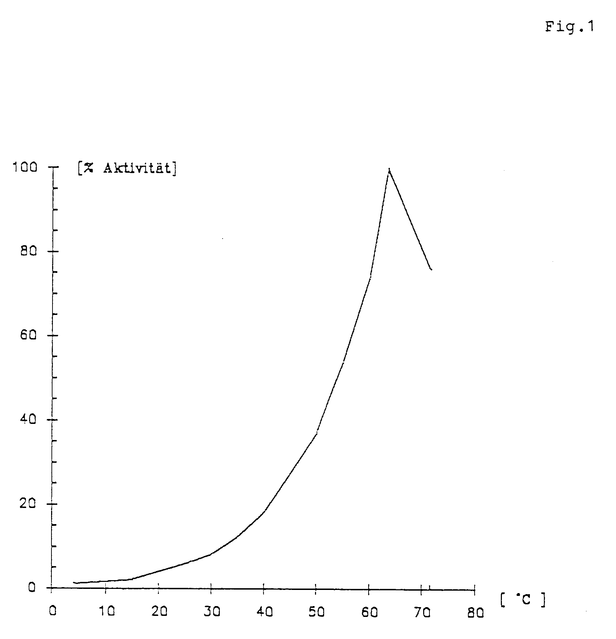

Das Temperatur-Optimum der in den Kulturüberständen enthaltenen Proteasen wurde im Bereich von 40 bis 72 °C bestimmt. Die Ergebnisse sind in Tabelle 2 sowie in der Figur 1 dargestellt.

Das Temperatur-Optimum der Protease aus Bacillus sp. MF12 DSM 6845 liegt bei 64 °C.

Tabelle 2

| Temperatur-Optimum der alkalischen Protease aus Bacillus sp.MF12 DSM 6845 | | | | | |
|---|---|---|---|---|---|
| Aktivität in % in Abhängigkeit von der Temperatur in °C | | | | | |
| 40 °C | 50 °C | 55 °C | 60 °C | 64 °C | 72 °C |
| 18 % | 37 % | 53 % | 74 % | 100 % | 76 % |

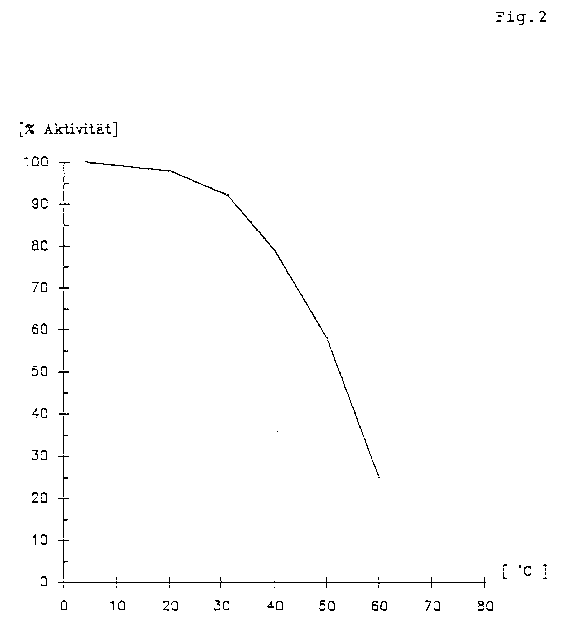

Zur Bestimmung der Temperatur-Stabilität wurden der proteasehaltige Überstand für 15 Minuten bei verschiedenen Temperaturen inkubiert und anschließend die Restaktivität bestimmt. Die Ergebnisse sind in der Tabelle 3 sowie in der Figur 2 dargestellt.

Die Protease aus Bacillus sp. MF12 DSM 6845 ist bis 31 °C stabil (Restaktivität > 90 %) und zeigt nach 15-minütiger Inkubation bei 50 °C noch eine Restaktivität von 58 %.

Tabelle 3

| Temperatur [°C] | proteolytische Restaktivität in % nach 15-minütiger Inkubation bei verschiedenen Temperaturen |
|---|---|
| 4 | 100 |
| 20 | 98 |
| 31 | 92 |
| 40 | 79 |
| 50 | 58 |
| 60 | 25 |

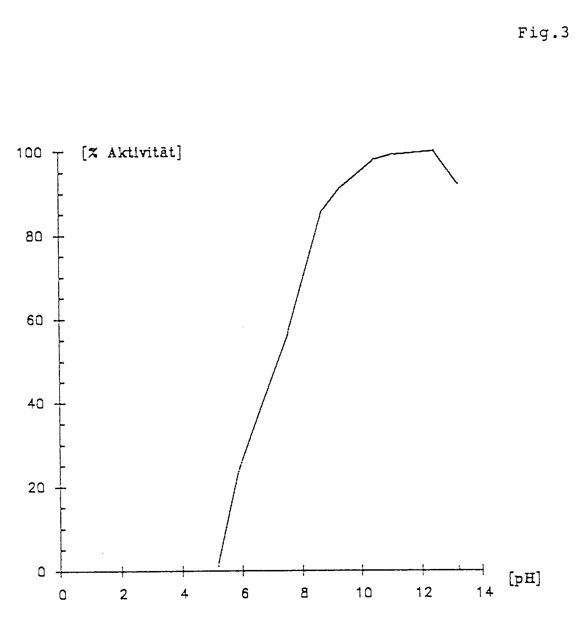

Zur Bestimmung des pH-Optimums der Protease aus Bacillus sp. MF12 DSM 6845 wurde die Aktivität bei verschiedenen pH-Werten bestimmt. Für die Einstellung der pH-Werte wurde im pH-Bereich 5,0 bis 7,0 Phosphat- (0,1 M), im pH-Bereich 7,0 bis 9,0 Tris-HCl- (0,1 M) und im pH-Bereich 9,0 bis 13,0 Glycin-NaOH-Puffer (0,1 M) verwendet. Die Werte der Aktivitätsbestimmung sind in Figur 3 dargestellt.

Das pH-Optimum der alkalischen Protease aus Bacillus sp. MF12 DSM 6845 liegt um pH 12. Bei pH 13 ist die Aktivität noch > 90 %.

Zur Untersuchung der pH-Stabilität wurden die Protease aus Bacillus sp. MF12 für 24 Stunden in Puffern verschiedenen pH-Wertes bei 4 °C inkubiert.

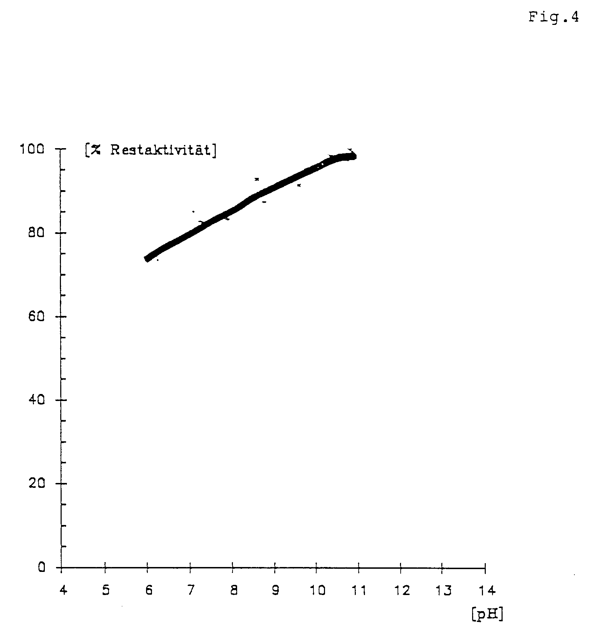

Anschließend wurde die Restaktivität der Proteasen bestimmt. Für den pH-Bereich von 5 bis 7,1 wurde Phosphat-Puffer (0,1 M), für den pH-Bereich von 7,5 bis 9 Tris-HCL-Pfuffer (Tris(hydroxymethyl)amino-methan-Puffer), (0,1 M) und für den pH-Bereich von 9 bis 12,1 Glycin/Natriumhydroxid-Puffer (0,1 M) verwendet. Das Ergebnis ist in der Figur 4 dargestellt.

Das Enzym aus Bacillus sp. MF12 DSM 6845 besitzt nach der 24-stündigen Inkubation im gesamten pH-Bereich noch mindestens 70 % Restaktivität und ist um pH 11 völlig stabil.

Beispiel 4

Waschleistung der Protease MF12 aus Bacillus sp. MF12 (DSM 6845).

Die Waschwirksamkeit der erfindungsgemäßen Protease MF12 wurde in Waschversuchen ermittelt.

Beispiel 4a

Für die Anwendung in Waschmittel wurde die Waschleistung der erfindungsgemäßen Protease durch Waschversuche am Testgewebe EY-PC (Eigelb/Tusche-Anschmutzung auf Polyester-Baumwolle-Mischgewebeeigene Herstellung) in Laborwaschmaschinen (Typ: Polycolor) bestimmt. Hierzu wurden das Testgewebe mit 6 g/l einer Waschmittelbasisformulierung, die 18,4 % Zeolith, 7,3 % $Na_2CO_3$, 4,8 % lineares Alkylbenzolsulfonat, 3,3 % Nonionics, 3,3 % Seife, 0,1 % Entschäumer, 1,5 % Carboxymethylcellulose, 0,15 % optischen Aufheller, 3,8 % Natrium-di-silikat, 25 % Perborat, 1,5 % TAED und 30,85 % $Na_2SO_4$ enthielt, nach Zusatz (5000 DU/l) der zu untersuchenden Protease mit Wasser von 15 °dH gewaschen. Der pH-Wert der Waschlauge betrug pH 10,3. Gewaschen wurde im Temperaturbereich von 15 °C bis 60 °C für 45 min (2 °C/min; 22,5 min Haltezeit).

Die enzymhaltige Waschmittellösung wirkte in einem rotierenden Probengefäß, das über ein Wasserbad entsprechend dem Temperaturprogramm gesteuert wurde, auf das Testgewebe ein. Nach dem Waschvorgang wurde das Testgewebe zweimal mit Leitungswasser gespült und anschließend gebügelt.

Die Waschleistung wurde durch Messung der Reflektion des gewaschenen Testgewebes bestimmt. Zum Vergleich wurde ebenfalls die Reflektion des trockenen, nur mit der Waschmittelbasisformulierung gewaschenen Testgewebes ermittelt.

Ohne Zusatz der erfindungsgemäßen Protease wurde eine Reflektion von 41,2, bei Zusatz der erfindungsgemäßen Protease zur Waschmittelbasisformulierung eine Reflektion des Testgewebes von 51,9 bestimmt.

Beispiel 4b

Die Untersuchung der Waschwirksamkeit der Protease MF12 wurde wie im Beispiel 4a beschrieben durchgeführt. Es wurde jedoch als Testgewebe ein mit Blut, Milch und Tusche angeschmutztes Polyester-Baumwolle-Mischgewebe (EMPA 117, bezogen von der Eidgenössischen Materialprüfungsanstalt, St. Gallen, Schweiz) verwendet. Die Waschmittelbasisformulierung war ein handelsübliches Pulvervollwaschmittel (pH 10,4).

Nach dem Waschen des Testgewebes mit enzymhaltiger Waschmittellösung wurde eine Reflektion von 59,0 gemessen. Wurde ein Testgewebe nur mit der Waschmittelbasisformulierung gewaschen, wurde lediglich eine Reflektion von 35,1 ermittelt.

Beispiel 4c

Die Untersuchung der Waschwirksamkeit der Protease MF12 wurde wie im Beispiel 4b beschrieben durchgeführt. Es wurde jedoch als Testgewebe ein mit Eigelb und Tusche angeschmutztes Polyester-Baumwolle-Mischgewebe verwendet.

Nach dem Waschen des Testgewebes mit enzymhaltiger Waschmittellösung wurde für das Testgewebe eine Reflektion von 51,9 gemessen. Wurde ein Testgewebe nur mit der Waschmittelbasisformulierung gewaschen, wurde für die Reflektion lediglich ein Wert von 41,3 ermittelt.

In allen durchgeführten Waschversuchen wurde für das unter Zusatz der erfindungsgemäßen Protease gewaschenen Testgewebe eine deutlich höhere Reflektion als bei nur mit der Waschmittelbasisformulierung

EP 0 577 973 A2

gewaschenen Testgewebe gemessen. Dies belegt die sehr gute Waschwirksamkeit der erfindungsgemäßen Protease, wobei auch die unterschiedlichen Proteinverschmutzungen problemlos beim Waschgang aus dem Gewebe entfernt werden.

**Patentansprüche**

1.  Alkalische Bacillus-Protease mit einem pH-Optimum im pH-Bereich von etwa pH 9,5 bis 13,0 und einem Temperatur-Optimum im Bereich von etwa 55 bis 65 °C, erhältlich durch Kultivierung von Bacillus sp. MF12 DSM 6845.

2.  Alkalische Bacillus-Protease gemäß Anspruch 1 mit folgenden Eigenschaften:
    (1) Wirkung: Abbau von Proteinen und Peptiden;
    (2) pH-Optimum: etwa bei pH-Werten von 9,5 bis 13,0
    (3) pH-Stabilität: bei pH-Werten von 9,5 bis 11,0 erweist sich die Protease als völlig stabil;
    (4) Temperatur-Optimum: etwa 64 °C;
    (5) Temperatur-Stabilität: durch Inkubation der Protease bei Temperaturen bis 30 °C für 15 Minuten wird die Protease nicht wesentlich in ihrer Aktivität beeinträchtigt; nach 15 Minuten Inkubation bei 40 °C beträgt die Restaktivität der Protease wenigstens 75 %.

3.  Zusammensetzungen zum Waschen, Reinigen oder Geschirrspülen, enthaltend eine alkalische Bacillus-Protease gemäß einem der Ansprüche 1 bis 2.

4.  Zusammensetzungen nach Anspruch 3, dadurch gekennzeichnet, daß sie die alkalische Bacillus-Protease in Gegenwart eines oder mehrerer von in Wasch- und Reinigungsmitteln üblichen Enzymen aus der Gruppe der Proteasen, Lipasen, Pektinasen, Amylasen, Nukleasen, Oxidoreduktasen und Cellulasen enthalten.

5.  Zusammensetzungen nach Anspruch 4, dadurch gekennzeichnet, daß sie die alkalische Bacillus-Protease in Gegenwart einer zweiten Protease enthalten.

6.  Zusammensetzungen nach einem der Ansprüche 3 bis 5, dadurch gekennzeichnet, daß sie die alkalische Bacillus-Protease in einer Formulierung für niedrige Anwendungstemperaturen bis etwa 60 °C enthalten.

7.  Verfahren zur Herstellung der alkalischen Bacillus-Protease gemäß einem der Ansprüche 1 bis 2, dadurch gekennzeichnet, daß man einen unter der Nummer DSM 6845 hinterlegten Bacillus sp. MF12 kultiviert und nachfolgend aus dem Kulturüberstand die gebildete alkalische Protease isoliert.

8.  Unter der Nummer DSM 6845 hinterlegter Bacillus sp. MF12.

9

Fig.1

Fig.2

[% Aktivität]

Fig.3

EP 0 577 973 A2

Fig.4

13